# EUROPÄISCHE PATENTSCHRIFT

(11) **EP 4 465 031 B1**
(45) Veröffentlichungstag und Bekanntmachung des Hinweises auf die Patenterteilung: **17.06.2026**
(21) Anmeldenummer: 23020542.9
(22) Anmeldetag: 07.12.2023
(51) Int. Cl.: G01N 25/18

(54) **VORRICHTUNG ZUR ERMITTLUNG DES WÄRMEDURCHLASSWIDERSTANDES EINES BAUKÖRPERS**
DEVICE FOR DETERMINING THE THERMAL RESISTANCE OF A COMPONENT
DISPOSITIF POUR DÉTERMINER LA RÉSISTANCE THERMIQUE D'UN CORPS DE CONSTRUCTION

(30) Priorität: 17.05.2023 DE 202023102715 U
(43) Veröffentlichungstag der Anmeldung: 20.11.2024
(73) Patentinhaber: Quade, Markus, 35428 Langgöns (DE)
(72) Erfinder: Quade, Markus, 35428 Langgöns (DE); Quade, Noah, 35428 Langgöns (DE)
(74) Vertreter: Körner, Volkmar Horst

(56) Entgegenhaltungen:
- WO-A1-85/04479
- DE-C- 965 447
- US-A1- 2014 286 373

## Beschreibung

Die Erfindung betrifft eine Vorrichtung zur Ermittlung des Wärmedurchlasswiderstandes eines Baukörpers, mit zumindest einer zur Anordnung auf einer Wandseite des Baukörpers vorgesehenen Messeinheit, wobei die Messeinheit Sensorelemente zur Ermittlung von physikalischen Werten der Wandseite aufweist und dass die zumindest eine Messeinheit gegen die Wandseite andrückbar ist.

Solche Vorrichtungen werden bei einem "Messgerät zur Baustoffprüfung" - einem Wandprüfstand nach DIN "Wärmetechnisches Verhalten von Gebäuden - Messung des Wärmedurchlasswiderstandes, Heizkastenverfahren mit dem Wärmestromesser - Mauerwerk, Deutsche Fassung EN 1934:1998" eingesetzt. Bei dieser Messung wird der Baukörper mit den Maßen L x H x B (1,50 x 1,50 x [0,16m bis 0,45m]) zwischen zwei halbschalenförmige Messkammern gestellt, wobei eine der Messkammern warm und die andere Messkammer kalt temperiert werden. Diese Temperaturdifferenz erzeugt einen Wärmestrom durch die Wand. Dieser Wärmestrom sowie die Oberflächentemperaturen werden zur Berechnung der Wärmeleitfähigkeit benötigt. Die Oberflächentemperaturen der Wand werden mit Temperatursensoren und der Wärmestrom mit einer Wärmestrommessplatte ermittelt.

Die US 2014/0286373 A1 offenbart eine Vorrichtung und ein Verfahren zur Messung eines statischen und eines dynamischen Wärmestroms und einer Wärmekapazität eines Wandabschnitts eines Bauwerks. Die Vorrichtung weist ein thermisch abgeschirmtes Gehäuse und einen thermoelektrischen Sensor und ein Vorspannelement auf.

Aus der WO 85/04479 A1 ist eine Einrichtung zur Ermittlung des Wärmedurchlasswiderstandes an Baukörpern bekannt geworden, bei dem eine auf einer Wandseite angeordnete Messeinheit mittels einer am Bauwerk abstützbaren Gelenkanordnung befestigt ist. Dies setzt jedoch ein ausreichend stabiles Bauwerk voraus. Zudem sind Wandseiten nicht immer zuverlässig eben, so dass fehlerhafte Messungen entstehen können.

Weiterhin ist aus der DE 965 447 eine Vorrichtung zur Prüfung der Wärmedurchlässigkeit für Wände bekannt geworden. Die Vorrichtung hat zwei gegeneinander gegenüberliegende Seiten verfahrbare Prüfkammern, welche über Dichtungspolster gegenüber der Wand abgedichtet sind. Ein Wärmedurchflussmesser ist an einem Leitblech befestigt.

Der Erfindung liegt das Problem zugrunde, eine Vorrichtung der eingangs genannten Art so weiterzubilden, dass sie besonders einfach handhabbar ist und genaue Messungen sicherstellt.

Dieses Problem wird erfindungsgemäß gelöst durch eine mit Druck beaufschlagbare Kammer zum Andrücken der zumindest einen Messeinheit gegen die eine Wandseite.

Durch diese Gestaltung wird der zuverlässige Kontakt der zumindest einen Messeinheit gegen die Wandseite durch den Druck in der Kammer erzeugt, so dass die Sensorelemente mit dem Druck der Kammer zuverlässig gegen die Wandseite gepresst sind. Hierdurch liegen die Sensorelemente zuverlässig an der Wandseite an, so dass besonders genaue Messungen gewährleistet sind. Dank der Erfindung ist die Vorrichtung besonders einfach handhabbar, weil die Druckbeaufschlagung der Kammer und das Druckablassen einfach steuerbar ist.

Die Vorrichtung könnte beispielsweise einen Pneumatikzylinder zur Anpressung der Messeinheit gegen die eine Wandseite aufweisen. Die Vorrichtung ist jedoch gemäß einer anderen vorteilhaften Weiterbildung der Erfindung konstruktiv besonders einfach aufgebaut, wenn die zumindest eine Messeinheit auf einer beweglichen Wandung der mit Druck beaufschlagbaren Kammer angeordnet ist.

Die Anbringung der Vorrichtung an den Baukörper gestaltet sich gemäß einer anderen vorteilhaften Weiterbildung der Erfindung besonders einfach, wenn in einer Grundstellung die Messeinheit von der vorgesehenen Position der Wandseite entfernt ist.

Die Messeinheit könnte beispielsweise auf einem von dem Druck in der Kammer verschieblichen Kolben angeordnet sein. Die Vorrichtung gestaltet sich jedoch gemäß einer anderen vorteilhaften Weiterbildung der Erfindung konstruktiv besonders einfach, wenn die zumindest eine Messeinheit auf gegenüber der mit Druck beaufschlagten Kammer über eine Membrane abgedichtet ist. Zudem lässt sich die Membrane mit einer Eigenspannung ausstatten, so dass bei einem Druckausgleich der Kammer mit der Umgebung die zumindest eine Messeinheit in die Grundstellung bewegbar ist.

Unebenheiten der Wandseiten lassen sich gemäß einer anderen vorteilhaften Weiterbildung der Erfindung einfach ausgleichen, wenn die zumindest eine Messeinheit auf einer flexiblen Matte angeordnet ist, wobei die flexible Matte auf einer Seite den Druck der Kammer und auf der anderen Seite dem Umgebungsdruck oder dem Gegendruck der Wandseite ausgesetzt ist. Hierdurch kann sich die flexible Matte fest an die Oberfläche der Wandseite anschmiegen und damit die zumindest eine Messeinheit fest an die Wandseite anpressen.

Zur weiteren konstruktiven Vereinfachung der Vorrichtung trägt es gemäß einer anderen vorteilhaften Weiterbildung der Erfindung bei, wenn die mit Druck beaufschlagbare Kammer als halbschalenförmige Messkammer ausgebildet ist und die halbschalenförmige Messkammer gegenüber der zumindest einen Messeinheit abgedichtet ist.

Die Anpressung der Sensorelemente gegen die Wandseite gestaltet sich gemäß einer anderen vorteilhaften Weiterbildung der Erfindung besonders einfach, wenn die zumindest eine Kammer einen Druckluftanschluss zur Erzeugung und Ablassung des Drucks hat. Der maximale Druck lässt sich mit einem Überdruckventil besonders einfach begrenzen.

Die Steuerung der Anpressung der Sensorelemente gegen die Wandseite gestaltet sich gemäß einer anderen vorteilhaften Weiterbildung der Erfindung besonders einfach, wenn die zumindest eine Kammer einen Druckluftsensor zur Erfassung des Drucks in der Kammer hat. Im einfachsten Fall ist der Druckluftsensor und ein Verdichter zur Erzeugung des Drucks mit einer Steuereinheit verbunden, so dass zum Start der Messung ein voreingestellter Druck in der Kammer erzeugt und am Ende der Messung abgelassen wird.

Zur Erhöhung der Genauigkeit der Ermittlung des Wärmedurchlasswiderstandes und zur Ermittlung weiterer Werte trägt es gemäß einer anderen vorteilhaften Weiterbildung der Erfindung bei, wenn auf jeder Wandseite jeweils eine Messeinheit angeordnet ist. Solche Messeinheiten können beispielsweise Temperatursensoren und eine Wärmemessplatte oder nur Temperatursensoren aufweisen.

Die Bewegungen der beiden Messeinheiten könnten beispielsweise miteinander gekoppelt sein. Eine solche Koppelung benötigt jedoch einen baulichen Aufwand und eine unmittelbare Verbindung der beiden Wandseiten. Zur weiteren Vereinfachung des konstruktiven Aufbaus der Vorrichtung trägt es gemäß einer anderen vorteilhaften Weiterbildung der Erfindung bei, wenn die Messeinheiten jeweils auf einer beweglichen Wandung einer mit Druck beaufschlagbaren Kammer angeordnet sind.

Die Erfindung lässt zahlreiche Ausführungsformen zu. Zur weiteren Verdeutlichung ihres Grundprinzips sind mehrere davon in der Zeichnung dargestellt und werden nachfolgend beschrieben. Diese zeigt in
- Fig. 1: eine Vorrichtung zur Ermittlung des Wärmedurchlasswiderstandes eines Baukörpers,
- Fig. 2: eine weitere Ausführungsform der Vorrichtung zur Ermittlung des Wärmedurchlasswiderstandes eines Baukörpers in einer Grundstellung,
- Fig. 3: die Vorrichtung aus Figur 2 in einer Messstellung.

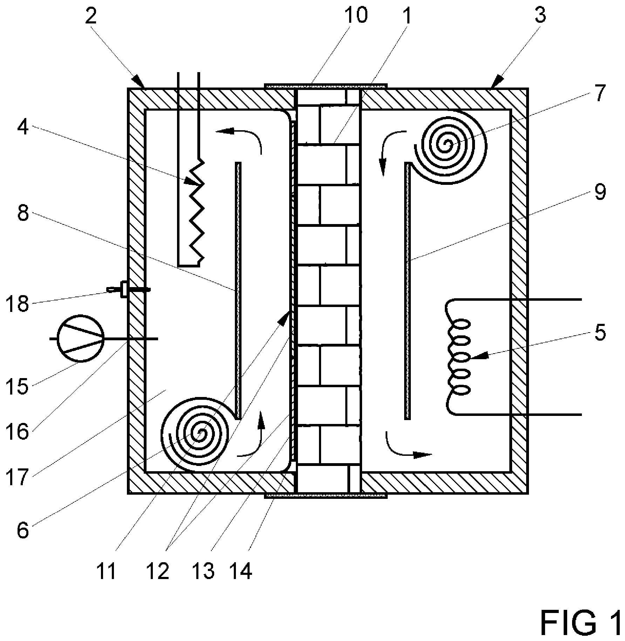

Figur 1 zeigt eine Vorrichtung zur Ermittlung des Wärmedurchlasswiderstandes eines Baukörpers 1 mit zwei halbschalenförmigen Messkammern 2, 3. Eine erste Messkammer 2 ist mit einer elektrischen Heizeinrichtung 4 als warme Seite ausgebildet. Die zweite Messkammer 3 hat eine Kühlschlange 5 und ist damit als kalte Seite ausgebildet. Beide Messkammern 2, 3 weisen jeweils ein Umluftgebläse 6, 7 mit schematisch dargestellten Luftleitblechen 8, 9 auf. Die Strömungen der Luft sind mit Pfeilen gekennzeichnet. Die beiden Messkammern 2, 3 und die Ränder des Baukörpers 1 sind mittels Seitenflächendämmung 10 isoliert. Ebenso sind die Wände der Messkammern 2, 3 wärmeisoliert. Bei dem Baukörper 1 handelt es sich um eine aufgemauerte und verputzte Wand mit den Abmessungen 1,50 x 1,50 m. Die Wanddicke beträgt in der Regel 0,16 bis 0,45m.

Die erste Messkammer 2 hat eine Messeinheit 11 mit Sensorelementen 12, wie einem Wärmestrommesser und/oder Thermoelemente. Die Messeinheit 11 ist auf einer flexiblen Matte 13 angeordnet und über Membrane 14 gegenüber Wänden der ersten Messkammer abgedichtet. Die flexible Matte 13 ist aus einem gummielastischen Material gefertigt und bildet eine auf die Wandseite des Baukörpers 1 hin und von dieser weg bewegliche Wandung. Die erste Messkammer 2 hat eine mit Druck von einem Verdichter 15 über einen Druckluftanschluss 16 beaufschlagte Kammer 17 und einen Drucksensor 18. Mit dem Verdichter 15 wird zur Messung des Wärmedurchlasswiderstandes in der Kammer 17 ein vorgesehener Druck erzeugt und damit die Messeinheit 11 gegen die eine Wandseite des Baukörpers 1 vorgespannt. Durch die Flexibilität der Matte 13 schmiegen sich die Sensorelemente 12 der Messeinheit 11 an die Wandseite und stellen einen zuverlässigen Kontakt her.

Die eigentliche Messung des Wärmedurchlasswiderstandes ist in der Norm DIN EN 1934 geregelt, so dass zur Offenbarung darauf verwiesen wird.

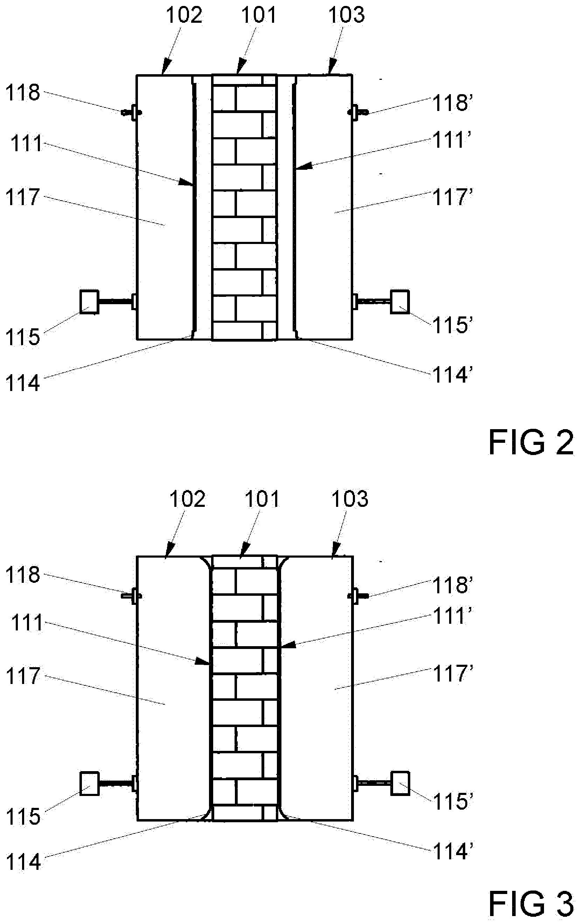

Die Figuren 2 und 3 zeigen eine weitere Ausführungsform einer Vorrichtung zur Ermittlung des Wärmedurchlasswiderstandes eines Baukörpers 101 mit zwei Messkammern 102, 103. Die Vorrichtung hat in jeder der Messkammern eine Messeinheit 111, 111'. Beide Messeinheiten 111, 111' sind über Membrane 114, 114' gegenüber den Messkammern 102, 103 abgedichtet und haben wie zu Figur 1 beschrieben auf einer flexiblen Matte angeordnete Sensorelemente. Die Messkammern 102, 103 weisen jeweils Kammern 117, 117' auf, welche von Verdichtern 115, 115' über Druckluftanschlüsse mit Druck beaufschlagbar sind. Drucksensoren 118, 118' in jeder Kammer 117, 117' erfassen den jeweiligen Druck.

In Figur 2 ist die Vorrichtung in einer Grundstellung dargestellt. In dieser Grundstellung herrscht in den Kammern 117, 117' der Umgebungsdruck, so dass durch die Eigenspannung der Membrane 114, 114' die Messeinheiten 111, 111' von dem Wandseiten des Baukörpers 101 entfernt sind.

Figur 3 zeigt die Vorrichtung während der Messung des Wärmedurchlasswiderstandes des Baukörpers 101. In den Kammern 117, 117' herrscht ein höherer Druck als in der Umgebung, so dass die Messeinheiten 111, 111' gegen die Wandseiten des Baukörpers 101 vorgespannt sind.

Zur Vereinfachung der Zeichnung sind in den Figuren 2 und 3 aus Figur 1 bekannte Heizelemente, Kühlschlangen und Gebläse nicht dargestellt. Diese Bauteile sind, wie in Figur 1 beschrieben, ebenfalls in den Messkammern 102, 103 angeordnet.

## Patentansprüche

1. Vorrichtung zur Ermittlung des Wärmedurchlasswiderstandes eines Baukörpers (1, 101), mit zumindest einer zur Anordnung auf einer Wandseite des Baukörpers (1, 101) vorgesehenen Messeinheit (11, 111, 111'), wobei die Messeinheit (11, 111, 111') Sensorelemente (12) zur Ermittlung von physikalischen Werten der Wandseite aufweist und dass die zumindest eine Messeinheit (11, 111, 111') gegen die Wandseite andrückbar ist, **gekennzeichnet durch** eine mit Druck beaufschlagbare Kammer (17, 117, 117') zum Andrücken der zumindest einen Messeinheit (11, 111, 111') gegen die eine Wandseite.

2. Vorrichtung nach Anspruch 1, **dadurch gekennzeichnet, dass** die zumindest eine Messeinheit (11, 111, 111') auf einer beweglichen Wandung der mit Druck beaufschlagbaren Kammer (17, 117, 117') angeordnet ist.

3. Vorrichtung nach Anspruch 1 oder 2, **dadurch gekennzeichnet, dass** in einer Grundstellung die Messeinheit (11, 111, 111') von der vorgesehenen Position der Wandseite entfernt ist.

4. Vorrichtung nach zumindest einem der Ansprüche 1 bis 3, **dadurch gekennzeichnet, dass** die zumindest eine Messeinheit (11, 111, 111') auf gegenüber der mit Druck beaufschlagten Kammer (17, 117, 117') über eine Membrane abgedichtet ist.

5. Vorrichtung nach zumindest einem der Ansprüche 1 bis 4, **dadurch gekennzeichnet, dass** die zumindest eine Messeinheit (11, 111, 111') auf einer flexiblen Matte angeordnet ist, wobei die flexible Matte auf einer Seite den Druck der Kammer (17, 117, 117') und auf der anderen Seite dem Umgebungsdruck oder dem Gegendruck der Wandseite ausgesetzt ist.

6. Vorrichtung nach zumindest einem der Ansprüche 1 bis 5, **dadurch gekennzeichnet, dass** die mit Druck beaufschlagbare Kammer (17, 117, 117') als halbschalenförmige Messkammer (2, 3, 102, 103) ausgebildet ist und die halbschalenförmige Messkammer (2, 3, 102, 103) gegenüber der zumindest einen Messeinheit (11, 111, 111') abgedichtet ist.

7. Vorrichtung nach zumindest einem der Ansprüche 1 bis 6, **dadurch gekennzeichnet, dass** die zumindest eine Kammer (17, 117, 117') einen Druckluftanschluss (16) zur Erzeugung und Ablassung des Drucks hat.

8. Vorrichtung nach zumindest einem der Ansprüche 1 bis 7, **dadurch gekennzeichnet, dass** auf die zumindest eine Kammer (17, 117, 117') einen Druckluftsensor (18) zur Erfassung des Drucks in der Kammer (17, 117, 117') hat.

9. Vorrichtung nach zumindest einem der Ansprüche 1 bis 8, **dadurch gekennzeichnet, dass** auf jeder Wandseite jeweils eine Messeinheit (111, 111') angeordnet ist.

10. Vorrichtung nach Anspruch 9, **dadurch gekennzeichnet, dass** die Messeinheiten (111, 111') jeweils auf einer beweglichen Wandung einer mit Druck beaufschlagbaren Kammer (117, 117') angeordnet sind.

## Claims

1. Device for determining the thermal resistance of a component (1, 101), with at least one measuring unit (11, 111, 111') intended for arrangement on a wall side of the component (1, 101), wherein the measuring unit (11, 111, 111') has sensor elements (12) for determining physical values of the wall side, and in that the at least one measuring unit (11, 111, 111') can be pressed against the wall side, **characterized by** a pressurizable chamber (17, 117, 117') for pressing the at least one measuring unit (11, 111, 111') against the one wall side.

2. Device according to claim 1, **characterized in that** the at least one measuring unit (11, 111, 111') is arranged on a movable wall of the pressurizable chamber (17, 117, 117').

3. Device according to claim 1 or 2, **characterized in that,** in a base position, the measuring unit (11, 111, 111') is removed from the intended position of the wall side.

4. Device according to at least one of claims 1 to 3, **characterized in that** the at least one measuring unit (11, 111, 111') is sealed off from the pressurized chamber (17, 117, 117') by a membrane.

5. Device according to at least one of claims 1 to 4, **characterized in that** the at least one measuring unit (11, 111, 111') is arranged on a flexible mat, wherein the flexible mat is exposed on one side to the pressure of the chamber (17, 117, 117') and on the other side to ambient pressure or the counterpressure of the wall side.

6. Device according to at least one of claims 1 to 5, **characterized in that** the pressurizable chamber (17, 117, 117') is in the form of a half-shell-shaped measuring chamber (2, 3, 102, 103) and the half-shell-shaped measuring chamber (2, 3, 102, 103) is sealed off from the at least one measuring unit (11, 111, 111').

7. Device according to at least one of claims 1 to 6, **characterized in that** the at least one chamber (17, 117, 117') has a compressed air connection (16) for generating and releasing the pressure.

8. Device according to at least one of claims 1 to 7, **characterized in that** the at least one chamber (17, 117, 117') has a compressed air sensor (18) for detecting the pressure in the chamber (17, 117, 117').

9. Device according to at least one of claims 1 to 8, **characterized in that** a measuring unit (111, 111') is arranged on each wall side.

10. Device according to claim 9, **characterized in that** the measuring units (111, 111') are each arranged on a movable wall of a pressurizable chamber (117, 117').

## Revendications

1. Dispositif de détermination de la résistance à la conductibilité thermique d'un corps de construction (1, 101) avec au moins une unité de mesure (11, 111, 111') prévue pour l'agencement sur un côté de paroi du corps de construction (1, 101), dans lequel l'unité de mesure (11, 111, 111') présente des éléments de capteur (12) pour la détermination de valeurs physiques du côté de paroi et en ce que l'au moins une unité de mesure (11, 111, 111') peut être pressée contre le côté de paroi, **caractérisé par** une chambre pouvant être alimentée en pression (17, 117, 117') pour la pression de l'au moins une unité de mesure (11, 111, 111') contre l'un côté de paroi.

2. Dispositif selon la revendication 1, **caractérisé en ce que** l'au moins une unité de mesure (11, 111, 111') est agencée sur une paroi mobile de la chambre (17, 117, 117') pouvant être alimentée en pression.

3. Dispositif selon la revendication 1 ou 2, **caractérisé en ce que** dans une position de base, l'unité de mesure (11, 111, 111') est retirée de la position prévue du côté de paroi.

4. Dispositif selon au moins l'une quelconque des revendications 1 à 3, **caractérisé en ce que** l'au moins une unité de mesure (11, 111, 111') est étanchéifiée par rapport à la chambre (17, 117, 117') pouvant être alimentée en pression par le biais d'une membrane.

5. Dispositif selon au moins l'une quelconque des revendications 1 à 4, **caractérisé en ce que** l'au moins une unité de mesure (11, 111, 111') est agencée sur un tapis flexible, dans lequel le tapis flexible est soumis d'un côté à la pression de la chambre (17, 117, 117') et de l'autre côté à la pression ambiante ou à la contre-pression du côté de paroi.

6. Dispositif selon au moins l'une quelconque des revendications 1 à 5, **caractérisé en ce que** la chambre (17, 117, 117') pouvant être alimentée en pression est configurée comme une chambre de mesure (2, 3, 102, 103) en forme de demi-coque et la chambre de mesure (2, 3, 102, 103) en forme de demi-coque est étanchéifiée par rapport à l'au moins une unité de mesure (11, 111, 111').

7. Dispositif selon au moins l'une quelconque des revendications 1 à 6, **caractérisé en ce que** l'au moins une chambre (17, 117, 117') présente un raccord d'air comprimé (16) pour générer et évacuer la pression.

8. Dispositif selon au moins l'une quelconque des revendications 1 à 7, **caractérisé en ce que** l'au moins une chambre (17, 117, 117') présente un capteur d'air comprimé (18) pour détecter la pression dans la chambre (17, 117, 117').

9. Dispositif selon au moins l'une quelconque des revendications 1 à 8, **caractérisé en ce qu'**une unité de mesure (111, 111') est agencée respectivement sur chaque côté de paroi.

10. Dispositif selon la revendication 9, **caractérisé en ce que** les unités de mesure (111, 111') sont agencées respectivement sur une paroi mobile d'une chambre (117, 117') pouvant être alimentée en pression.
